# FASCICULE DE BREVET EUROPEEN

(11) **EP 1 222 636 B1**
(45) Date de publication et mention de la délivrance du brevet: **17.03.2004**
(21) Numéro de dépôt: 00962642.5
(22) Date de dépôt: 14.09.2000
(51) Int. Cl.: G06T 11/00

(54) **RECONSTITUTION STATISTIQUE DE SURFACES EN TROIS DIMENSIONS**
DREIDIMENSIONALE STATISTISCHE FLÄCHENREKONSTRUKTION
THREE-DIMENSIONAL STATISTIC RECONSTRUCTION OF SURFACES

(30) Priorité: 17.09.1999 FR 9911848
(43) Date de publication de la demande: 17.07.2002
(73) Titulaire: UNIVERSITE JOSEPH FOURIER, 38041 Grenoble Cédex 9 (FR)
(72) Inventeur: FLEUTE, Markus, F-38400 Saint Martin d'Heres (FR); LAVALLEE, Stéphane, F-38000 Grenoble (FR); DESBAT, Laurent, F-38000 Grenoble (FR)
(74) Mandataire: de Beaumont, Michel
(86) Numéro de dépôt international: PCT/FR2000/002546
(87) Numéro de publication internationale: WO 2001/022368

(56) Documents cités:
- LAVALLEE S ET AL: "RECOVERING THE POSITION AND ORIENTATION OF FREE-FORM OBJECTS FROM IMAGE CONTOURS USING 3D DISTANCE MAPS" IEEE TRANSACTIONS ON PATTERN ANALYSIS AND MACHINE INTELLIGENCE,US,IEEE INC. NEW YORK, vol. 17, no. 4, 1 avril 1995 (1995-04-01), pages 378-390, XP000499568 ISSN: 0162-8828
- FLEUTE, M.; LAVALL E, S.: "Building a complete surface model from sparse data using statistical shape models : application to computer assisted knee surgery" MEDICAL IMAGE COMPUTING AND COMPUTER-ASSISTED INTERVENTION - MICCAI'98 PROC., - 1 octobre 1998 (1998-10-01) XP000913649 Cambridge, MA, USA cité dans la demande
- GUEZIEC,A., KAZANZIDES,P.; ET AL.: "Anatomy-based registration of CT-scan and intraoperative X-Ray images for guiding a surgical robot" IEEE TRANS. MED. IMAG., vol. 17, no. 5, 1 octobre 1998 (1998-10-01), pages 715-728, XP002140303 cité dans la demande
- LAVALLEE S ET AL: "MATCHING OF MEDICAL IMAGES FOR COMPUTED AND ROBOT ASSISTED SURGERY" PROCEEDINGS OF THE ANNUAL INTERNATIONAL CONFERENCE OF THE ENGINEERING IN MEDICINE AND BIOLOGY SOCIETY,US,NEW YORK, IEEE, vol. CONF. 13, 1991, pages 39-40, XP000347734

## Description

La présente invention concerne la reconstitution d'images en trois dimensions et, plus particulièrement, la reconstitution des contours surfaciques d'une image à partir de vues, même incomplètes, en deux dimensions.

Un exemple d'application de la présente invention est la reconstitution d'images représentant des éléments osseux à partir d'images en deux dimensions prises, par exemple, aux rayons X. De telles images peuvent servir, par exemple, à simuler une intervention chirurgicale en permettant au praticien de pré-visualiser les positions respectives des os au niveau d'une articulation, par exemple, en cas de plastie ligamentaire ou de mise en place de prothèse.

Aujourd'hui, pour permettre au praticien de visualiser une image en trois dimensions à partir d'images en deux dimensions, on est contraint d'utiliser un scanner à rayons X effectuant des tomographies. A partir de ces tomographies, on sait reconstituer une image en trois dimensions. Soit il s'agit d'une technologie dite de tomographies en trois dimensions dans laquelle un grand nombre de vues (de l'ordre de 200) sont prises au moyen d'un scanner à rayons X selon différentes positions, et les contours de l'os sont alors reconstitués par traitement d'image à partir de ces vues au scanner. Soit il s'agit d'une technologie dite de tomographies en deux dimensions dans laquelle un grand nombre de coupes sont prises au moyen d'un scanner à rayons X transversalement à l'os. On peut alors reconstituer l'allure et la structure de l'os.

La technique du scanner donne de bons résultats mais est d'une mise en oeuvre lourde et coûteuse. En effet, l'utilisation d'un scanner permet d'obtenir un ensemble d'images en deux dimensions fournissant non seulement des informations sur le contour mais également sur l'intérieur de l'os. Or, dans de nombreuses applications, seule la connaissance du contour surfacique de l'os ou de l'objet est nécessaire.

L'article "Recovering the position and orientation of free-form object from image contours using 3D distance maps" de S. Lavallée, publié dans IEEE Transactions on Pattern Analysis and Machine Intelligence, volume 17, N° 4, en avril 1995, décrit un procédé de reconstitution de la position et de l'orientation d'un objet dont on connaît le modèle 3D. Cette reconstitution s'effectue à partir d'images en deux dimensions.

Un autre exemple d'application de la présente invention est la reconstitution d'ossements incomplets, par exemple, en archéologie. Une reconstitution d'images en trois dimensions peut permettre de retrouver de façon quasi-parfaite la forme originelle de l'os même si celui-ci est découvert de façon incomplète. Dans une telle application, des problèmes similaires à ceux exposés ci-dessus en relation avec la simulation d'interventions chirurgicales se posent. En particulier, il est souvent utile de connaître la forme de l'os sans avoir à se préoccuper de sa structure interne.

Un autre inconvénient des techniques connues est qu'elles imposent une dose de radiations importante pour le patient ce qui n'est pas souhaitable. Si cet inconvénient est moins sensible dans le domaine de l'archéologie où c'est le coût qui prédomine, il est particulièrement gênant dans la simulation d'interventions chirurgicales.

La présente invention s'applique plus particulièrement à la reconstitution d'images se rapportant à des objets identifiés, c'est-à-dire, dont on connaît à l'avance la forme générale. Par exemple, pour un os, il faut au préalable décider de quel os il s'agit.

La présente invention vise à proposer un nouveau procédé de reconstitution d'images en trois dimensions qui pallie les inconvénients des techniques connues. L'invention vise, en particulier, à proposer une solution qui ne nécessite pas l'emploi coûteux d'un scanner à rayons X.

La présente invention vise également à proposer une solution qui soit compatible avec une exposition minimale aux rayons X ou équivalents.

L'invention vise en outre à minimiser le nombre de vues en deux dimensions nécessaires pour reconstituer l'image en trois dimensions.

Pour atteindre ces objets, la présente invention prévoit un procédé de reconstitution d'une image en trois dimensions représentant les contours surfaciques d'au moins un objet, à partir d'au moins une vue en deux dimensions de cet objet prise aux rayons X, caractérisé en ce qu'il consiste à :
déterminer la position de la source de prise de vues dans un référentiel de référence ;
sélectionner un modèle prédéfini constituant une forme moyenne de l'objet ; et
de façon itérative jusqu'à ce que les contours du modèle soient tels que les écarts, entre des rayons de rétroprojection des contours de l'image en deux dimensions depuis la source et la surface du modèle, soient minimaux :
sélectionner une orientation et une position du modèle dans le référentiel de référence, puis
sélectionner une déformation du modèle pour modifier ses contours en trois dimensions.

Selon un mode de réalisation de la présente invention, le modèle est obtenu à partir d'une population d'objets pour laquelle on recherche la correspondance statistique commune à tous les objets pour déterminer une forme moyenne et les déformations principales par rapport à cette forme moyenne, de façon à disposer d'au moins un modèle statistique.

Selon un mode de réalisation de la présente invention, les étapes de sélection itératives consistent à faire subir au modèle statistique, successivement, une transformation rigide modifiant sa position et/ou son orientation et une déformation non-rigide modifiant ses contours surfaciques.

Selon un mode de réalisation de la présente invention, les contours de l'image en deux dimensions sont obtenus automatiquement en projetant le modèle dans le plan de l'image en deux dimensions, et en déformant les contours projetés de façon à les faire coïncider avec les points de fort gradient en niveaux de gris de l'image en deux dimensions.

Selon un mode de réalisation de la présente invention, la détermination automatique des contours de l'image en deux dimensions est effectuée de façon itérative, chaque itération étant intercalée entre deux itérations successives des étapes de sélection.

Selon un mode de réalisation de la présente invention, on détermine, dans le référentiel de référence, des coordonnées en trois dimensions de points de l'objet, de façon à disposer de points de référence supplémentaires pour les étapes de sélection itératives de position, orientation, et déformation.

Selon un mode de réalisation de la présente invention, on utilise plusieurs images en deux dimensions pour lesquelles les positions respectives de la source de prise de vues sont toutes déterminées dans le référentiel de référence, et on effectue les étapes de sélections itératives en tenant compte des rayons de rétroprojection des contours de toutes les images en deux dimensions.

Selon un mode de réalisation de la présente invention, le nombre d'images utilisé est fonction de la précision souhaitée.

Selon un mode de réalisation de la présente invention, la surface du modèle est constituée d'éléments de triangle, lesdits écarts étant mesurés par rapport à des points de certaines arêtes constituant des générateurs du contour en trois dimensions.

Selon un mode de réalisation de la présente invention, le procédé est appliqué à la reconstitution des contours surfaciques de plusieurs objets liés entre eux par des relations de transformation rigide et/ou élastique.

Selon un mode de réalisation de la présente invention, le procédé est appliqué à la reconstitution d'images d'os.

L'invention concerne également un système de traitement d'images, comportant des moyens pour la mise en oeuvre du procédé de reconstitution d'images en trois dimensions.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
la figure 1 représente un mode de réalisation d'un système de reconstitution d'images en trois dimensions à partir d'un dispositif de prise de vues en deux dimensions selon la présente invention ;
la figure 2A illustre la prise d'une vue en deux dimensions pour la mise en oeuvre du procédé de reconstitution d'images en trois dimensions selon la présente invention ;
la figure 2B illustre la prise de trois vues en deux dimensions pour la mise en oeuvre du procédé de reconstitution selon la présente invention ;
les figures 3A, 3B et 3C illustrent trois étapes caractéristiques du procédé selon un mode de réalisation de la présente invention ;
la figure 4 illustre un mode de détermination de générateurs de contour selon la présente invention ; et
la figure 5 illustre un mode de détermination préféré selon l'invention de l'écart entre un modèle en trois dimensions et une vue en deux dimensions.

Les mêmes éléments ont été désignés par les mêmes références aux différentes figures qui ne sont pas à l'échelle. Pour des raisons de clarté, seuls les éléments qui sont nécessaires à la compréhension de l'invention ont été représentés aux figures et seront décrits par la suite. En particulier, les moyens de traitement d'images comprenant les calculateurs informatiques n'ont pas été représentés et ne seront pas décrits de façon détaillée, car leur réalisation est à la portée de l'homme du métier à partir des indications fonctionnelles données ci-après.

Une caractéristique de la présente invention est de rechercher la position et l'orientation d'un objet à reconstituer, et dont on connaît au moins une vue en deux dimensions, à partir d'une base de données contenant des modèles de cet objet. Quand plusieurs vues en deux dimensions sont utilisées, ces vues sont toutes référencées dans un même référentiel. Ainsi, l'invention concerne la reconstitution d'une image représentant un objet déjà identifié et dont on peut disposer de modèles de taille et/ou de forme différentes.

Une caractéristique d'un mode de réalisation préféré de la présente invention est d'utiliser au moins un modèle statistique déformable, établi à partir de la base de données, pour reconstituer la forme en trois dimensions de l'objet. Ainsi, l'invention prévoit de définir, avant toute reconstitution, une base de données contenant des modèles en trois dimensions de l'objet à reconstituer ou, de préférence, un ou plusieurs modèles statistiques déformables à partir de cette base de données.

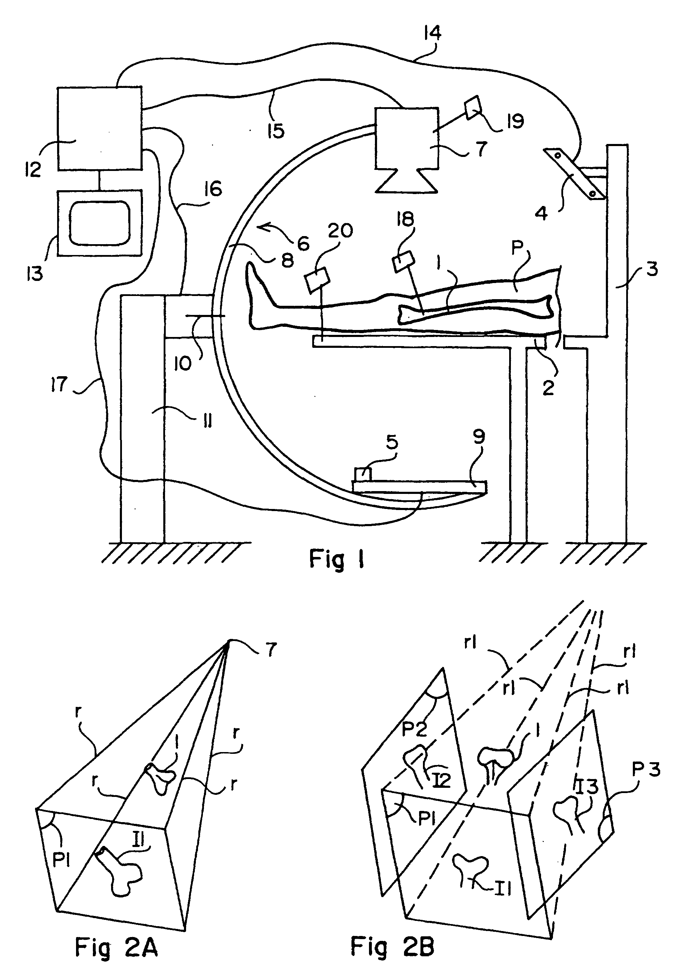

La figure 1 représente une vue schématique d'un système de reconstitution d'images en trois dimensions selon un mode de réalisation de la présente invention. L'exemple de la figure 1 concerne la reconstitution de l'image d'un os 1 à partir de radiographies en deux dimensions. En figure 1, on a représenté schématiquement et partiellement le corps d'un patient p dont la jambe contient l'os 1 que l'on souhaite visualiser. La jambe du patient p (donc l'os 1) est, par exemple, posé sur une table 2. Une potence 3 porte un dispositif 4 de localisation en trois dimensions qui peut être optique, magnétique, mécanique ou ultrasonore et qui repère la position et l'orientation de multiples capteurs-émetteurs constitués, par exemple, de diodes infrarouges, de réflecteurs, d'émetteurs magnétiques, à ultrasons, etc. Un tel localisateur est parfaitement connu et ne sera pas détaillé plus avant. On notera simplement que, pour garantir une localisation correcte du patient (de l'os 1), celui-ci est généralement également équipé d'un capteur-émetteur 18 détectable par le localisateur 4. En effet, selon l'invention, toutes les vues doivent pouvoir être exploitées dans un même référentiel (de référence), qui est associé à l'objet que l'on reconstitue.

Plusieurs capteurs-émetteurs peuvent être fixés sur le système de radiologie, proche de la source 7 (capteur-émetteur 19) ou proche d'un détecteur d'image 9 (capteur-émetteur 5), de façon à repérer la position du système de radiologie par rapport au référentiel de référence du capteur-émetteur 18.

Dans certains cas, les capteurs-émetteurs 5 et 19 sont difficilement repérables en raison de leur éloignement ou de la présence d'objets parasites dans le champ de mesure du localisateur 4. Dans ce cas, un capteur-émetteur 20 peut être installé sur la table 2 dans le champ de mesure du localisateur 4. On positionne alors le système radiologique dans de bonnes conditions de mesure et on repère les positions des capteur-émetteurs 5 et 19 par rapport au capteur-émetteur 20 une fois pour toutes (on ne renouvelle cette étape que si on bouge le système de radiologie dans son ensemble).

Le système radiologique étant équipé de codeurs angulaires sur ses axes, comme cela sera détaillé plus loin, les changements de position relative du système radiologique sont mesurés à l'aide de ces codeurs et peuvent ainsi être reportés dans le référentiel du capteur-émetteur 20. Pour chaque prise de vue, on mesure la relation géométrique entre les capteurs-émetteurs 20 et 18 et, par ce biais, on reporte la position du système radiologique dans le référentiel de référence du capteur-émetteur 18. Ainsi, toutes les radiographies sont calibrées dans le référentiel du capteur-émetteur 18 qui peut être mobile.

Le dispositif 6 est, par exemple, constitué d'une source 7 à rayons x portée par une première extrémité d'un bras 8 en demi-cercle dont l'autre extrémité est destinée à recevoir le film 9 d'impression de la radiographie, ou un capteur électronique équivalent tel qu'un amplificateur de brillance, ou un détecteur plat au silicium amorphe. La relation entre le bras 8 et la table 2 est telle que cette dernière se trouve entre la source 7 et le capteur 9. Le bras 8 est monté à rotation autour d'un axe 10, motorisé ou à déplacement manuel, et porté par une potence 11. L'ensemble de prise de vues en deux dimensions peut ainsi tourner autour de l'os 1 pour effectuer le nombre désiré de radiographies de celui-ci. Le cas échéant, le localisateur optique 4 peut être associé à un dispositif de codage angulaire de la position de l'axe 10.

L'ensemble est piloté par un système informatique, par exemple, un ordinateur 12 associé, de préférence, à un écran de visualisation 13. A la figure 1, on a symbolisé par des liaisons unifilaires 14, 15, 16 et 17, les bus informatiques d'échange de signaux électriques de commande et de données entre l'ordinateur 12 et, respectivement, le localisateur 4, la source 7, le moteur ou le codeur optionnel de l'axe 10 et le capteur 9.

Les figures 2A et 2B illustrent deux modes de prises de vues selon deux modes de réalisation de la présente invention. Aux figures 2A et 2B, les vues en deux dimensions ont été représentées par les plans respectifs dans lesquelles elles sont prises, c'est-à-dire par l'allure de la surface du capteur 9 (figure 1) lors des prises. Cela correspond aux images radiographiques en deux dimensions récupérées par le système informatique.

La figure 2A représente le cas d'une seule prise de vue dans un plan P1, fournissant une image Il de l'os 1. La position de la source 7 a été symbolisée par un point où convergent des rayons r de rétroprojection des quatre coins du plan P1.

La figure 2B représente le cas d'une triple prise de vues dans des plans P1, P2 et P3, fournissant trois images I1, I2 et I3 en deux dimensions de l'os 1. En figure 2B, la position de la source n'est pas constante, celle-ci est différente pour chaque prise de vue. Toutefois, grâce au localisateur 4, toutes les positions de la source sont connues dans le référentiel de référence. Par soucis de clarté, seuls les rayons r1 des quatre coins du plan P1 dans la position de l'image I1 ont été représentés.

On notera que la source de prises de vues peut subir, entre deux images en deux dimensions, d'autres mouvements que dans un même plan comme illustré par les figures 1 et 2B. En d'autres termes, le bras 8 du système de prises de vues 6 peut présenter plus de deux degrés de liberté, dont chacun pourra être muni d'un dispositif de codage angulaire. Selon un mode de réalisation préféré, le bras 8 est monté sur deux axes de rotation horizontal et vertical et un axe de translation vertical.

On notera également que les images en deux dimensions peuvent n'être que partielles. Par exemple, dans l'application aux radiographies, celles-ci peuvent être interprétées par l'opérateur pour valider les contours des vues en deux dimensions à prendre en compte pour la reconstitution. Une telle interprétation n'est pas gênante en raison du faible nombre de vues en deux dimensions nécessaire selon l'invention (moins de dix en général). La validation des contours dans le système informatique peut être effectuée, par exemple, au moyen d'une souris, d'un crayon optique, d'un écran tactile, ou équivalent, de façon classique pour un enregistrement de contour sur une image en niveaux de gris.

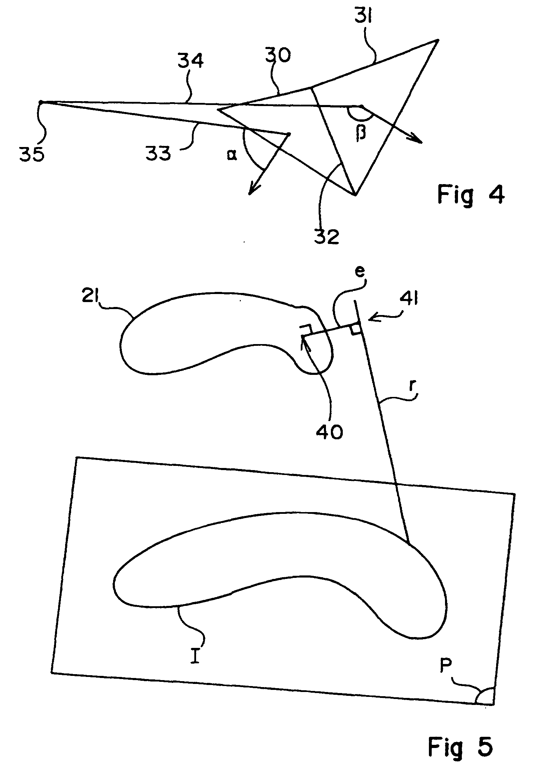

Selon un autre mode de réalisation préféré, la détermination des contours sur les images radiographiques est automatisée en mettant en oeuvre un procédé dit de recalage en deux dimensions. Un tel procédé consiste à déterminer le contour de façon automatique par analyse des gradients les plus forts en niveaux de gris. Ce procédé est décrit, par exemple, dans la thèse de Gelu Ionescu, présentée publiquement le 4 décembre 1998 à l'Université Joseph Fourier de Grenoble (France), et ayant pour titre "Segmentation et recalage d'images échographiques par utilisation de connaissances physiologiques et morphologiques". Selon l'invention, on met en oeuvre ce procédé de recalage en deux dimensions en combinaison avec une projection, sur l'image en deux dimensions, du modèle statistique déformable de l'objet dont on souhaite reconstituer les contours surfaciques en trois dimensions. On effectue une analyse itérative d'un contour projeté du modèle sur l'image en deux dimensions jusqu'à obtenir, après plusieurs passes (par exemple de 3 à 10), une identité entre le contour du modèle projeté qui sera décrit ci-après et dont l'orientation et la déformation auront été adaptés par le procédé de l'invention, et le contour déterminé par analyse des gradients les plus forts en niveaux de gris. Cette combinaison sera mieux comprise par la suite en relation avec la figure 5.

Pour simplifier la présente description, on considérera ci-après que les contours des vues en deux dimensions sont connus. En pratique, les étapes du procédé de l'invention seront effectuées en boucle, y compris le recalage en deux dimensions, jusqu'à obtenir un résultat satisfaisant.

Selon l'invention, une fois que la ou les vues en deux dimensions ont été obtenues, il reste à déterminer, par recherche de correspondance dans la base de données, la forme et la taille de l'objet (ici, l'os) en trois dimensions.

Pour ce faire, l'invention prévoit de rechercher le modèle pour lesquelles les distances séparant les contours de chaque vue en deux dimensions et la surface du modèle sont minimales.

Dans le mode de réalisation préféré où on utilise un modèle statistique déformable (définissant ainsi une famille de modèles) de l'objet à reconstituer, on recherche à s'approcher le plus possible, par déformation itérative de ce modèle, de la forme dont les contours sont tels que, comme on le verra par la suite en relation avec la figure 5, les rayons de rétroprojection s'appuyant sur les points des contours de l'image (ou des images) en deux dimensions depuis la source (ou depuis leurs sources respectives) sont tous tangentiels à la surface du modèle, étant entendu que les différents contours en deux dimensions sont repérables dans le référentiel de référence.

La réalisation du modèle statistique déformable en lui même ne fait pas l'objet de la présente invention et est parfaitement classique. On se bornera à rappeler que la réalisation d'un tel modèle fait généralement appel à une recherche d'une forme moyenne d'une population d'objets de même type (par exemple, des fémurs) constituant la base de données, suivie d'une analyse en composantes principales pour déterminer les déformations principales (essentielles) à appliquer à cette forme moyenne et obtenir ainsi le modèle statistique.

Une méthode de détermination d'un modèle statistique à partir d'une famille d'échantillons est décrite, par exemple, dans l'article "Building a Complete Surface Model from Sparse Data Using Statistical Shape Models: Application to Computer Assisted Knee Surgery" de Markus Fleute et Stéphane Lavallée, paru dans MEDICAL IMAGE COMPUTING AND COMPUTER-ASSISTED INTERVENTION - MICCAI'98, Springer-Verlag LNCS Series, pages 880-887, en Octobre 1998, et dont le contenu est incorporé ici par référence. On notera que la précision de la reconstruction des contours surfaciques opérée par l'invention dépend des échantillons utilisés pour construire la base de données du modèle statistique. Par exemple, si un modèle statistique de fémur est créé à partir d'une population d'échantillons ayant des formes normales (sans pathologie), on pourra reconstituer avec précision des formes normales mais la précision sera limitée si le fémur radiographié a une forme pathologique. Par contre, si le modèle statistique est créé à partir d'une grande population d'échantillons contenant à la fois des formes normales et des formes pathologiques, on pourra reconstituer, avec précision, des objets normaux et pathologiques.

Selon l'invention, une fois que l'on dispose des images en deux dimensions, on recherche le cas échéant à quel modèle contenu dans la base de données ces images se rapprochent le plus (par exemple, si plusieurs types d'os ou si plusieurs modèles statistiques d'un même os sont disponibles), puis on applique des transformations à ce modèle pour s'approcher le plus possible d'une image en trois dimensions, telle que les rayons de rétroprojection gentiels aux surfaces de l'image en trois dimensions, placée sur le trajet de ces rayons et dont ils sont respectivement les plus proches.

De préférence, on commence par faire subir au modèle initial au repos, c'est-à-dire correspondant à la forme moyenne, une transformation dite rigide, c'est-à-dire, ne touchant qu'à son orientation et sa translation dans l'espace. Puis, quand on a obtenu la position pour laquelle les rayons de rétroprojection sont tous à une distance minimale des contours du modèle initial, on fait subir une déformation non-rigide au modèle, c'est-à-dire que sans modifier son orientation, on modifie sa forme à partir des données contenues dans la base statistique en modifiant les coefficients des modes principaux du modèle, jusqu'à obtenir la forme dont les contours se rapprochent le plus des rayons de rétroprojection. Le cas échéant, le modèle est prépositionné de façon très approximative par l'opérateur par rapport aux vues en deux dimensions affichées à l'écran. On notera que les moyens (informatiques) de traitement d'images utilisés sont classiques dans leur structure et n'ont donc pas besoin d'être détaillés.

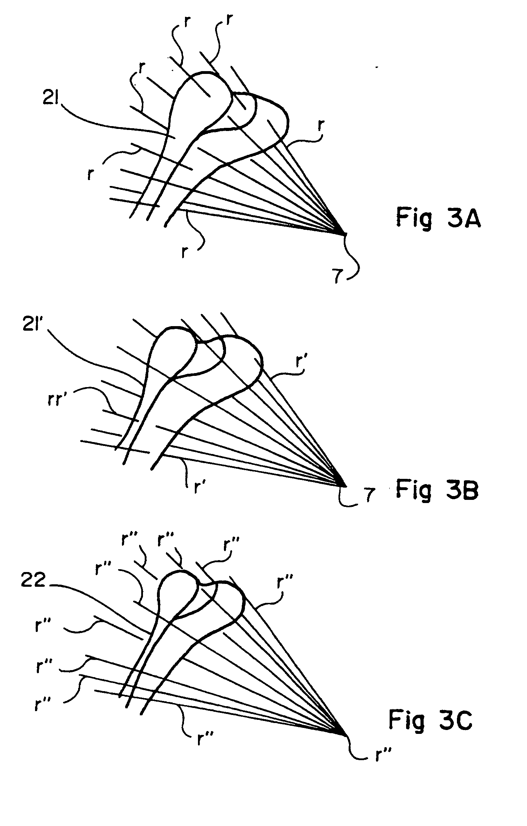

Les figures 3A à 3C illustrent ces deux étapes du procédé de l'invention. La figure 3A représente une vue en trois dimensions d'un modèle 21 avant toute déformation. Il s'agit, par exemple, d'un modèle statistique de l'invention positionné par le praticien dans une orientation approximative. La figure 3B représente le modèle 21' de la figure 3A à l'issue de l'étape d'orientation sans modification de forme. La figure 3C représente l'image 22 en trois dimensions résultant de la mise en oeuvre de l'invention, c'est-à-dire correspondant au modèle 21', déformé pour que les rayons de rétroprojection soient (idéalement) tangents à tous ses contours.

Par souci de simplification, un seul groupe de rayons r de rétroprojection depuis une source 7 ont été représentés aux figures 3A à 3C et l'écran de projection de l'image correspondante n'a pas été représenté. Comme il ressort des figures 3A à 3C, un grand nombre de rayons r de la figure 3A ne sont pas tangents aux contours du modèle 21. Le nombre de rayons de rétroprojection utilisé dépend de la précision souhaitée et des caractéristiques du modèle statistique, essentiellement, du nombre de points surfaciques choisis pour servir de référence lors de la définition de ce modèle statistique.

De la figure 3A à la figure 3B, la transformation est "rigide", c'est-à-dire que le modèle n'est pas déformé. Lors de cette transformation, le modèle 21 subit des translations et rotations afin d'obtenir des mesures d'écarts minimum entre chaque rayon de rétroprojection partant des points de contour de l'image et la surface de l'objet dans sa position courante. On utilise pour cela des méthodes mathématiques de recherche du minimum de la somme des carrés de ces écarts par rapport aux six paramètres (trois degrés de liberté en rotation et trois degrés de liberté en translation) définissant la transformation rigide recherchée. On obtient une configuration telle qu'illustrée par la figure 3B dans laquelle le modèle 21' a une position et orientation correcte mais une forme encore imparfaite. C'est pourquoi certain rayons r' ne sont pas tangents mais traversent le modèle.

De préférence, la position résultant de la transformation rigide (rotation, translation) est obtenue par une méthode (algorithme) dite du point le plus proche (Iterative Closest Point, ICP). Un exemple d'une telle méthode est décrit dans l'article "A Method for Registration of 3-D Shapes" de Paul J. Best et Neil D. McKay, paru dans IEEE TRANSACTIONS ON PATTERN ANALYSIS AND MACHINE INTELLIGENCE, vol. 14, NO. 2, en février 1992, et dont le contenu est incorporé ici par référence.

De la figure 3B à la figure 3C, la transformation est "non-rigide" (élastique) et consiste à déformer le modèle 21' sans changer son orientation jusqu'à obtenir l'image 22 en trois dimensions pour laquelle tous les rayons de rétroprojection r" sont à une distance minimale des contours (idéalement tangents). Cette transformation non-rigide est effectuée, par exemple et selon un premier mode de réalisation, en calculant les paramètres de déformation à partir d'un algorithme connu sous la dénomination anglo-saxonne "Down Hill Simplex". Cet algorithme a été décrit, par exemple, en 1965 par J.A. Nelder et R. Mead dans COMPUTER JOURNAL, vol. 7, pages 308-313, dont le contenu est incorporé ici par référence.

Selon un mode de réalisation préféré de l'invention, l'algorithme utilisé pour déterminer la transformation non-rigide du modèle statistique est basé sur la méthode de Levenberg-Marquardt. Le principe de cette méthode est de déterminer le minimum d'une fonction non-linéaire multidimensionnelle en utilisant les dérivées partielles de la fonction par rapport aux paramètres de déformation du modèle. Cette méthode a été décrite, par exemple, en 1963 par D.W. Marquardt dans JOURNAL OF THE SOCIETY FOR INDUSTRIAL AND APPLIED MATHEMATICS, vol. 11, pages 431-441, dont le contenu est incorporé ici par référence. La fonction dont on cherche le minimum est ici la somme des carrés des distances entre un ensemble de rayons de rétroprojection s'appuyant sur les points de contours et la surface du modèle. Les paramètres de la recherche du minimum sont les coefficients que l'on applique à chaque mode de déformation du modèle statistique, ainsi que cela est présenté dans l'article "Building a Complete Surface Model from Sparse Data Using Statistical Shape Models: Application to Computer Assisted Knee Surgery" de Markus Fleute et Stéphane Lavallée, déjà cité.

Cette méthode est appliquée et répétée à chaque position approchée jusqu'à ce qu'il ne soit plus possible de minimiser les écarts. On enchaîne ainsi un recalage rigide, un recalage élastique, un recalage rigide, un recalage élastique, etc. Une fois la forme en trois dimensions reconstituée, sa taille est bien entendu également connue dans la mesure où toutes les vues en deux dimensions ont des tailles connues, de même que la position, dans le référentiel de référence, du modèle par rapport aux sources. Le nombre de passes nécessaire dépend essentiellement du nombre de paramètres caractéristiques utilisés pour modéliser la déformation.

On notera que l'ordre dans lequel sont effectuées les deux étapes ci-dessus est important. En effet, si on commence par effectuer une déformation non-rigide, on va faire correspondre un modèle avec une mauvaise orientation qu'il sera très difficile à récupérer en raison de la déformation de forme subie. Toutefois, la minimisation des écarts peut être effectuée globalement en prenant en compte les paramètres rigides et les paramètres de déformation en même temps à chaque itération du processus mathématique de minimisation.

On notera que, à chaque étape itérative du procédé . dans laquelle on calcule les écarts entre les contours du modèle et les rayons de rétroprojection, de nouveaux points entrant dans la mesure sont déterminés côté modèle et/ou côté rayons de projection. Ceci constitue une distinction importante par rapport à des procédés connus de reconstitution d'images en trois dimensions dans lesquels les points de mesure sont les mêmes pour toutes les itérations.

Une autre caractéristique de la présente invention est que les évaluations des écarts minimaux ne sont pas nécessairement faites de façon exhaustive pour tous les éléments de surface du modèle. En effet, cela conduirait parfois à des calculs trop dispendieux même si le nombre d'éléments de surface peut être minimisé en utilisant des éléments triangulaires. Selon l'invention, les évaluations d'écarts sont faites par rapport à des lignes caractéristiques constituant des générateurs de contours du modèle. Cela minimise le nombre de points de mesure. Par exemple, un os de type fémur, défini par environ 5000 bords de surfaces triangulaires, est défini par environ 300 générateurs de contours.

De préférence, l'analyse effectuée par le procédé de l'invention ne touche que des générateurs de contour de l'image en trois dimensions. Ainsi, la surface du modèle statistique est, de préférence, formée d'éléments triangulaires dont certaines arêtes définissent des générateurs du contour. Un générateur de contour est défini par les arêtes (bords) des triangles qui, projetés sur un plan, définissent le contour (interne ou externe). Le recours aux générateurs de contours permet de réduire considérablement (par exemple, d'au moins un facteur 10) le nombre de points à chercher dans le modèle pour vérifier la correspondance avec l'image reconstituée.

La figure 4 illustre la définition d'un générateur de contour d'une image (modèle) en trois dimensions dont la surface est formée d'éléments de surface triangulaires tels que décrits, par exemple, dans l'article "Anatomy-based registration of ct-scan and intraoperative x-ray images for guiding a surgical robot" de A. Gueziec, paru dans IEEE TRANSACTIONS ON MEDICAL IMAGING, 17(5), pages 715-728, en octobre 1998, et dont le contenu est incorporé ici par référence.

La figure 4 représente, de façon simplifiée, deux triangles 30 et 31 définissant une portion de surface d'un modèle en trois dimensions et dont l'arête commune constitue une génératrice 32 du contour. Le calcul à effectuer pour déterminer si une arête constitue ou non une génératrice du contour consiste à calculer les angles α et β respectifs entre les normales aux surfaces des triangles 30 et 31 et les rayons 33 et 34 reliant le centre de ces surfaces au centre 35 de projection correspondant à la position de la source de rayons X. Si l'un des angles est inférieur à 90° tandis que l'autre est supérieur à 90°, leur arête commune 32 est alors une génératrice du contour.

La figure 5 illustre le type de mesure effectuée pour déterminer l'écart e entre un rayon r de rétroprojection et un point d'un générateur de contour. Cette figure représente, de façon schématique, une image I de l'objet dans un plan P et une forme 21 du modèle statistique placée sur le trajet des rayons de rétroprojection de l'image I jusqu'à la source (non représentée).

Pour chaque rayon de rétroprojection r, on cherche les points respectifs 41 et 40 du rayon de rétroprojection et d'un générateur de contour choisi parmi tous les générateurs, pour lequel l'écart e entre ces points est minimal.

Un avantage de la présente invention est qu'elle permet une reconstitution beaucoup plus rapide d'une image en trois dimensions par rapport aux techniques connues.

Un autre avantage de la présente invention est qu'elle permet un alignement correct du modèle même dans des zones où on ne dispose pas de données de projection par les images en deux dimensions (par exemple, certaines courbures internes).

On notera que le contour de surface pourra, le cas échéant, être affiné au moyen d'un palpeur mécanique, optique ou magnétique fournissant des coordonnées spatiales dans le même référentiel que les images en deux dimensions. On obtient alors des points supplémentaires qui peuvent être utilisés dans la recherche de l'image en trois dimensions. Le recours à un palpeur peut servir, par exemple, à diminuer le nombre de vues en deux dimensions nécessaires en donnant une information en trois dimensions.

On notera également que le procédé de l'invention peut s'appliquer à plusieurs contours surfaciques en trois dimensions constituant un ou plusieurs objets. Par exemple, on peut ainsi reconstituer simultanément la surface externe et la surface corticale interne d'un os. On peut également reconstituer la forme de plusieurs os participant à une articulation et visibles simultanément sur les images radiologiques (par exemple, le tibia et le fémur si on s'intéresse au genou), ou encore la forme de plusieurs fragments osseux d'un même os. Le modèle recherché doit, dans ce dernier cas, contenir les transformations rigides entre chaque contour surfacique en trois dimensions. Dans d'autre cas d'application à plusieurs objets, il faut bien entendu connaître les différents modèles statistiques et les éventuelle transformations rigides ou élastiques entre eux.

Bien entendu, la présente invention est susceptible de diverses variantes et modifications qui apparaîtront à l'homme de l'art. En particulier, le nombre de vues en deux dimensions devant être utilisé pour la reconstitution dépend de la précision souhaitée et de la complexité du modèle. Dans certains cas, une seule vue en deux dimensions pourra suffire. De plus, bien que l'invention ait été décrite en relation avec une source de rayons X mobile, celle-ci pourra être remplacée par plusieurs sources fixes, pourvu de respecter la contrainte d'obtenir des images en deux dimensions dans un même référentiel. En outre, la mise en oeuvre de l'invention fait bien entendu appel à des techniques de traitement numérique d'images dont la réalisation pratique est à la portée de l'homme du métier à partir des indications fonctionnelles données ci-dessus et dans les publications citées en références.

## Revendications

1. Procédé de reconstitution d'une image en trois dimensions représentant les contours surfaciques d'au moins un objet (1), à partir d'au moins une vue en deux dimensions de cet objet prise aux rayons X, **caractérisé en ce qu'**il consiste à :
déterminer la position de la source (7) de prise de vues dans un référentiel de référence ;
sélectionner au moins un modèle statistique définissant une forme moyenne de l'objet et ses déformations principales par rapport à cette forme moyenne, le modèle statistique étant calculé à partir d'une population d'objets de même type pour laquelle on recherche la correspondance statistique commune à tous les objets ; et
de façon itérative jusqu'à ce que les contours du modèle soient tels que les écarts, entre des rayons de rétroprojection des contours de l'image en deux dimensions depuis la source et la surface du modèle, soient minimaux, pour obtenir une correspondance entre le modèle et l'image :
sélectionner une orientation et une position du modèle dans le référentiel de référence en faisant subir au modèle statistique une transformation rigide modifiant sa position et/ou son orientation, puis
sélectionner une déformation du modèle en faisant subir au modèle statistique une déformation non rigide modifiant ses contours surfaciques.

2. Procédé selon la revendication 1, **caractérisé en ce que** les contours de l'image en deux dimensions sont obtenus automatiquement en projetant le modèle dans le plan de l'image en deux dimensions, et en déformant les contours projetés de façon à les faire coïncider avec les points de fort gradient en niveaux de gris de l'image en deux dimensions.

3. Procédé selon la revendication 2, **caractérisé en ce que** la détermination automatique des contours de l'image en deux dimensions est effectuée de façon itérative, chaque itération étant intercalée entre deux itérations successives des étapes de sélection.

4. Procédé selon l'une quelconque des revendications 1 à 3, **caractérisé en ce qu'**il consiste à déterminer, dans le référentiel de référence, des coordonnées en trois dimensions de points de l'objet, de façon à disposer de points de référence supplémentaires pour les étapes de sélection itératives de position, orientation, et déformation.

5. Procédé selon l'une quelconque des revendications 1 à 4, **caractérisé en ce qu'**il consiste à utiliser plusieurs images en deux dimensions pour lesquelles les positions respectives de la source de prise de vues sont toutes déterminées dans le référentiel de référence, et à effectuer les étapes de sélections itératives en tenant compte des rayons de rétroprojection des contours de toutes les images en deux dimensions.

6. Procédé selon la revendication 5, **caractérisé en ce que** le nombre d'images utilisé est fonction de la précision souhaitée.

7. Procédé selon l'une quelconque des revendications 1 à 6, **caractérisé en ce que** la surface du modèle est constituée d'éléments de triangle, lesdits écarts étant mesurés par rapport à des points de certaines arêtes constituant des générateurs du contour en trois dimensions.

8. Procédé selon l'une quelconque des revendications 1 à 7, **caractérisé en ce qu'**il est appliqué à la reconstitution des contours surfaciques de plusieurs objets liés entre eux par des relations de transformation rigide et/ou élastique.

9. Procédé selon l'une quelconque des revendications 1 à 8, **caractérisé en ce qu'**il est appliqué à la reconstitution d'images d'os.

10. Système de traitement d'images, **caractérisé en ce qu'**il comporte des moyens pour la mise en oeuvre du procédé selon l'une quelconque des revendications 1 à 9.

## Claims

1. A method for restoring a three-dimensional image representing the surface contours of at least one object (1), based on at least one two-dimensional X-ray view of this object, **characterized in that** it consists of:
determining the position of the shooting source (7) in a reference referential system;
selecting at least one statistical model defining an average shape (21) of the object and its main deformations with respect to this average shape, the statistical model being calculated from an object population of a same type for which the statistical correspondence common to all objects is searched; and
iteratively, until the contours of the model are such that the intervals between back-projection rays of the image contours in two dimensions from the source and the model surface are minimum, to obtain a correspondence between the model and the image:
selecting an orientation and a position of the model in the reference referential system by submitting the statistical model, successively, to a rigid transformation modifying its position and/or its orientation, then
selecting a deformation of the model to modify its contours in three dimensions, by submitting the statistical model to a non-rigid deformation modifying its surface contours.

2. The method of claim 1, **characterized in that** the image contours in two dimensions are automatically obtained by projecting the model in the image plane in two dimensions, and by deforming the projected contours to have them coincide with the points of strong grey level gradient of the two-dimensional image.

3. The method of claim 2, **characterized in that** the automatic determination of the image contours in two dimensions is performed iteratively, each iteration being interposed between two successive iterations of the selection steps.

4. The method of any of claims 1 to 3, **characterized in that** it consists of determining three-dimensional coordinates of points of the object in the reference referential system, to have additional reference points for the iterative position, orientation, and deformation selection steps.

5. The method of any of claims 1 to 4, **characterized in that** it consists of using several two-dimensional images for which the respective positions of the shooting source are all determined in the reference referential system, and of performing the iterative selection steps while taking account of the back-projection rays of the contours of all the two-dimensional images.

6. The method of claim 5, **characterized in that** the number of used images is a function of the desired accuracy.

7. The method of any of claims 1 to 6, **characterized in that** the model surface is formed of triangle elements, said intervals being measured with respect to points of given edges forming generators of the three-dimensional contour.

8. The method of any of claims 1 to 7, **characterized in that** it is applied to the restoring of the surface contours of several objects linked together by rigid and/or resilient transformation relations.

9. The method of any of claims 1 to 8, **characterized in that** it is applied to the restoring of bone images.

10. An image processing system, **characterized in that** it includes means for implementing the method of any of claims 1 to 9.

## Patentansprüche

1. Verfahren zur Rekonstitution eines die Oberflächenkonturen wenigstens eines Objekts (1) wiedergebenden Bildes in drei Dimensionen, ausgehend von wenigstens einer mit Röntgenstrahlung gemachten zweidimensionalen Aufnahme dieses Objekts, **dadurch gekennzeichnet, daß** das Verfahren umfaßt:
Bestimmen der Lage bzw. Stellung der Aufnahmestrahlungsquelle (7) in einem Bezugs-Referentialsystem:
Wählen wenigstens eines statistischen Modells, welches eine durchschnittliche mittlere Form des Objekts und seine Hauptdeformationen relativ bezüglich dieser mittleren durchschnittlichen Form definiert, wobei das statistische Modell ausgehend von einer Population von Objekten desselben Typs bestimmt wird, für welche man die sämtlichen Objekten gemeinsame statistische Korrespondenz-Entsprechung sucht; sowie
iterative Durchführung der folgenden Schritte, bis die Konturen solcher Art sind, daß die Abstände zwischen den Rückprojektionsstrahlen der Konturen des zweidimensionalen Bildes von der Strahlungsquelle und der Modelloberfläche minimal sind, zur Erlangung einer Korrespondenzentsprechung zwischen dem Modell und dem Bild:
Wählen einer Orientierung bzw. Ausrichtung und einer Stellung bzw. Lage des Modells in dem Bezugsreferentialsystem, indem man das statistische Modell einer starren Transformation unter Änderung seiner Stellung/Lage und/oder seiner Orientierung /Ausrichtung unterwirft, sodann
Wählen einer Deformation des Modells, indem man das statistische Modell einer nicht-starren Deformation unter Veränderung seiner Oberflächenkonturen unterwirft.

2. Verfahren nach Anspruch 1, **dadurch gekennzeichnet, daß** die Konturen des zweidimensionalen Bildes automatisch erhalten werden, indem man das Modell in die Ebene des zwei-dimensionalen Bildes projiziert und die projizierten Konturen so deformiert, daß sie mit den Punkten von starkem Grauwertgradient des zwei-dimensionalen Bildes zusammenfallen..

3. Verfahren nach Anspruch 2, **dadurch gekennzeichnet, daß** die automatische Bestimmung der Konturen des zweidimensionalen Bildes in iterativer Form erfolgt, wobei jede Iteration jeweils zwischen zwei aufeinanderfolgenden Iterationen der Wählverfahrensschritte zwischengeschaltet wird.

4. Verfahren nach einem der Ansprüche 1 bis 3, **dadurch gekennzeichnet, daß** es aus der Bestimmung dreidimensionaler Koordinaten von Punkten des Objekts im Referenz-Referentialsystem besteht, um über zusätzliche Referenzpunkte für die Verfahrensstufen iterativer Wahl von Lage/Stellung, Orientierung/Ausrichtung und Deformation zu verfügen.

5. Verfahren nach einem der Ansprüche 1 bis 4, **dadurch gekennzeichnet, daß** es aus der Verwendung mehrerer zweidimensionaler Bilder besteht, für welche die jeweiligen Lagen/Stellungen der Aufnahme-Strahlungsquellen sämtlich in dem Referenz-Referentialsystem bestimmt werden, sowie in der Durchführung der iterativen Wählschritte unter Berücksichtigung der Rückprojektionsstrahlen der Konturen aller zweidimensionalen Bilder.

6. Verfahren nach Anspruch 5, **dadurch gekennzeichnet, daß** die verwendete Bildzahl eine Funktion der gewünschten Genauigkeit ist.

7. Verfahren nach einem der Ansprüche 1 bis 6, **dadurch gekennzeichnet, daß** die Oberfläche des Modells von Dreieckselementen gebildet wird, wobei die genannten Abstände relativ bezüglich Punkten bestimmter Randkanten gemessen werden, welche Erzeugende der Kontur in drei Dimensionen bilden.

8. Verfahren nach einem der Ansprüche 1 bis 7, **dadurch gekennzeichnet, daß** das Verfahren auf die Rekonstitution der Oberflächenkonturen mehrerer Objekte angewandt wird, die untereinander durch Relationen starrer und oder elastischer Transformation verbunden sind.

9. Verfahren nach einem der Ansprüche 1 bis 8, **dadurch gekennzeichnet, daß** das Verfahren auf die Rekonstitution von Knochenbildern angewandt wird.

10. System zur Bildbe-bzw.―verarbeitung, **dadurch gekennzeichnet, daß** es Mittel zur Ausführung des Verfahrens nach einem der Ansprüche 1 bis 9 aufweist.
